Europäisches Patentamt

European Patent Office

Office européen des brevets

(19)

(11) Numéro de publication : **0 334 728 B1**

(12) **FASCICULE DE BREVET EUROPEEN**

(45) Date de publication du fascicule du brevet :
**21.07.93 Bulletin 93/29**

(51) Int. Cl.⁵ : **C12M 1/12,** C12P 7/06,
C12C 11/04, C12G 1/02

(21) Numéro de dépôt : **89400740.0**

(22) Date de dépôt : **16.03.89**

(54) **Procédé d'oxygenation controlée d'un moût de fermentation alcoolique, et installation correspondante.**

(30) Priorité : **22.03.88 FR 8803705**

(43) Date de publication de la demande :
**27.09.89 Bulletin 89/39**

(45) Mention de la délivrance du brevet :
**21.07.93 Bulletin 93/29**

(84) Etats contractants désignés :
**CH DE ES FR GR IT LI**

(56) Documents cités :
**EP-A- 0 103 988
EP-A- 0 160 442
WO-A-86/06094
GB-A- 1 518 261
GB-A- 2 197 341
BIOTECHNOLOGY AND BIOENGINEERING
SYMP., no. 17, 1986, pages 304-316, John
Wiley & Sons, Inc., New York, N.Y., US; G.T.
FRANK et al.: "An integrated bioreactorseparator: in situ recovery of fermentation
products by a novel membrane-based disper-
sion-free solvent extraction technique"**

(56) Documents cités :
**RESEARCH DISCLOSURE, no. 272, décembre
1986, page 738, résumé no. 27254, New York,
N.Y., US; "A method for supplying oxygen to a
fermentation liquor during mainly unaerobic
fermentation in a fermentor for achieving
growth of a microorganism contained in the
fermentation liquor"**

(73) Titulaire : **L'AIR LIQUIDE, SOCIETE ANONYME
POUR L'ETUDE ET L'EXPLOITATION DES
PROCEDES GEORGES CLAUDE
75, Quai d'Orsay
F-75321 Paris Cédex 07 (FR)**

(72) Inventeur : **Cutayar, Jacques-Marcel
9, rue Saint Pol Roux Le Bois de la Grille
F-78280 Guyancourt (FR)**
Inventeur : **Poillon, Dominique
46, rue du Moulin
F-91430 Igny (FR)**
Inventeur : **Cutayar, Sylvie
9, rue Saint Pol Roux Le Bois de la Grille
F-78280 Guyancourt (FR)**

(74) Mandataire : **Vesin, Jacques et al
L'AIR LIQUIDE 75 Quai d'Orsay
F-75321 Paris Cédex 07 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à la fermentation alcoolique. Elle s'applique notamment aux techniques de vinification, de brasserie, de cidrerie, de distillerie, etc...

Comme il est bien connu, la fermentation alcoolique est la réaction biologique au cours de laquelle les sucres à 6 atomes de carbone (hexoses) sont transformés en éthanol par des levures.

Une caractéristique de la fermentation alcoolique est que l'éthanol produit est inhibiteur de la réaction (rétroinhibition). Ceci se traduit par une diminution de plus en plus importante de la vitesse de production d'éthanol lorsque celui-ci s'accumule dans le moût de fermentation, ce phénomène se poursuivant jusqu'à l'arrêt complet de la réaction.

Il est important en fermentation alcoolique d'utiliser des levures présentant une bonne résistance à l'éthanol (alcoolorésistance), de façon à réduire le temps de fermentation (meilleure activité) et/ou à augmenter le degré alcoolique du moût en fin de fermentation.

Il a été montré que la résistance à l'alcool était proportionnelle à la concentration en stérols et en lipides insaturés dans les levures. Ces composés sont des constituants essentiels de leur membrane cellulaire et ne sont synthétisés et accumulés qu'en présence d'oxygène.

Lorsque la fermentation alcoolique est démarrée, l'oxygène initialement présent dans le moût est rapidement consommé, et les levures continuent alors à fermenter et à se développer en anaérobiose. On assiste alors à une utilisation et à une redistribution des stérols et des lipides insaturés (qui ne sont plus biosynthétisés) qui servent à l'élaboration des membranes des cellules filles. Il en résulte une baisse continuelle de leur concentration dans les levures, entraînant une sensibilité à l'éthanol de plus en plus importante allant jusqu'à l'inhibition totale de la fermentation (on estime que cette inhibition est effective au bout de 4 à 5 générations).

Pour éviter ou retarder ce phénomène, il est nécessaire d'oxygéner le moût de fermentation avant que l'oxygène dissous initialement présent soit entièrement consommé. Cette opération est en général effectuée par ce que les industriels appellent le "remontage des moûts", consistant à pomper le moût à la base de la cuve pour le refouler au sommet ; l'aération s'effectue alors par le ciel gazeux et permet d'atteindre une concentration en oxygène dissous de l'ordre de 4 à 5 mg/litre. Cette opération est généralement réalisée quotidiennement et pendant un temps réduit (quelques dizaines de minutes).

Cependant, il a été montré qu'une concentration en oxygène dissous trop importante pouvait altérer les qualités organoleptiques du vin :
- altération du goût : risques d'oxydation de certains composés (éthanol en acétaldéhyde voire en acide acétique,...) ;
- altération de la couleur : essentiellement pour les vins blancs, risques de brunissement enzymatique (action des polyphénoloxydases,...).

On peut admettre que dans la pratique, ces phénomènes apparaissent lorsque la concentration en oxygène dissous dépasse 0,1 à 0,5 mg/l suivant le moût traité.

Dans ces conditions, il peut se produire que la technique de remontage des moûts ne soit pas la plus adaptée, puisqu'elle n'offre aucun contrôle du transfert d'oxygène et donc ne peut permettre d'éviter les problèmes ci-dessus.

Pour mieux contrôler l'oxygénation du moût, il a été proposé d'effectuer un bullage d'une atmosphère appauvrie en oxygène (dilution d'air à l'azote) de façon à éviter toute suroxygénation locale au niveau de l'interface gaz/liquide. Toutefois, compte tenu d'une part de la faible concentration en oxygène dans le gaz d'aération (0,5 à 2%) et d'autre part de la demande en oxygène des levures, un tel système conduit à la mise en oeuvre de débits d'aération non négligeables entraînant inévitablement des pertes en éthanol.

L'invention a pour but de fournir une technique permettant d'obtenir une oxygénation bien contrôlée du moût sans perte consécutive d'éthanol.

A cet effet, l'invention a pour objet un procédé d'oxygénation contrôlée d'un moût de fermentation alcoolique, caractérisée en ce qu'on met le moût en contact avec une face d'une membrane perméable à l'oxygène; on met l'autre face de cette membrane en contact avec un gaz contenant de l'oxygène sous une pression partielle supérieure à la pression partielle en oxygène du liquide; on maintient constant le rapport de la pression du gaz à la pression du moût; et l'on régule l'oxygénation du moût en faisant varier la teneur en oxygène du gaz.

De préférence, on utilise comme gaz un mélange d'oxygène et d'un gaz neutre, notamment un mélange oxygène/azote.

L'invention a également pour objet une installation pour la mise en oeuvre d'un tel procédé. Cette installation de fermentation alcoolique, du type comprenant un fermenteur contenant un moût de fermentation, et des moyens d'oxygénation de ce moût, est caractérisée en ce que les moyens d'oxygénation comprennent un perméateur comportant une membrane perméable à l'oxygène; des moyens pour mettre une face de la membrane au contact du moût; et des moyens pour amener un gaz contenant de l'oxygène au contact de l'autre face de cette membrane, ces moyens d'amenée de gaz comprenant une source d'un gaz neutre, notamment d'azote, une source d'oxygène, des moyens de dosage et de mélange du gaz neutre et de l'oxygène, et un détendeur situé en aval des moyens de dosage et de mélange.

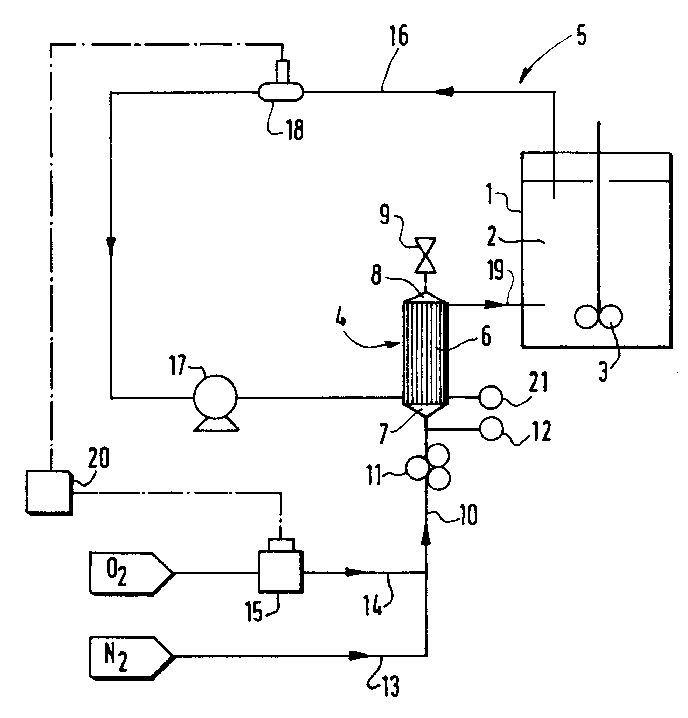

Un exemple de mise en oeuvre de l'invention va maintenant être décrit en regard du dessin annexé, sur lequel la figure unique représente schématiquement une installation de fermentation alcoolique conforme à l'invention.

L'installation représentée au dessin comprend essentiellement une cuve de fermentation ou fermenteur 1 contenant un moût de fermentation 2 et muni d'un agitateur 3, un perméateur 4 et un circuit 5 de circulation du moût entre le fermenteur 1 et le perméateur 4.

La perméateur 4 est constitué par au moins un module membranaire du type à fibres creuses. Ces fibres s'étendent, dans un carter 6, entre un collecteur d'entrée de gaz 7 et un collecteur de sortie de gaz 8. Chaque fibre est constituée d'une membrane microporeuse perméable à l'oxygène, par exemple formée de polypropylène ou d'une mince couche de silicone appliquée sur une couche-support macroporeuse. De tels modules de perméation sont disponibles dans le commerce, et l'on peut par exemple utiliser un module "ENKA modèle LM2P12" qui comporte 22 fibres creuses en polypropylène de 1,8 mm de diamètre extérieur et 1,2 mm de diamètre intérieur, la surface d'échange totale étant 0,03 m². 

Le collecteur de sortie 8 comporte une vanne 9 de mise à l'air, tandis que le collecteur d'entrée 7 est relié à une conduite 10 d'alimentation en gaz pourvue d'un détendeur 11 et, en aval de celui-ci, d'un manomètre 12. La conduite 10 est elle-même alimentée par une conduite d'azote 13 et par une conduite d'oxygène 14 équipée d'un débitmètre massique 15.

Le circuit 5 comprend une conduite 16 s'étendant du fermenteur au perméateur et équipée d'une pompe 17 et d'une sonde ampérométrique 18 de mesure de la concentration de l'oxygène dissous dans le moût. Cette sonde peut être par exemple la sonde connue sous la dénomination commerciale "ORBISPHERE". La conduite 16 aboutit à une extrémité du carter du perméateur 2, et une autre conduite 19 s'étend de l'autre extrémité de ce carter jusqu'au fermenteur.

Le débitmètre 15 est commandé par les signaux émis par la sonde 18, via un régulateur 20 du type PID, et un manomètre 21 mesure la pression $P_L$ du liquide dans le carter du perméateur, pression qui est fonction du débit de la pompe 17 pour un moût donné.

En fonctionnement, le moût circule dans le circuit 5, et un mélange oxygène/azote est envoyé dans le collecteur 7 du perméateur, et de là dans les fibres creuses, sous une pression $P_G$. On maintient constant le rapport $P_G/P_L$, légèrement au-dessous de la valeur critique conduisant à l'apparition de bulles dans le liquide. On peut alors contrôler le transfert d'oxygène en faisant varier uniquement la composition du gaz introduit dans le perméateur.

Pour cela, on choisit comme point de consigne de la sonde 18 une valeur d'environ 0,1 à 0,5 mg $O_2$/l, qui est la valeur au-delà de laquelle on peut considérer qu'existe une suroxygénation défavorable du moût. La sonde 18 régule ainsi le débitmètre 15 pour moduler le débit d'oxygène venant se mélanger au débit d'azote fourni par la conduite 13, lequel est supposé constant. Le mélange est ensuite détendu en 11 pour être délivré au perméateur à une pression constante en accord avec le rapport optimal $P_G/P_L$.

Ainsi, de l'oxygène est en permanence transféré de façon contrôlée dans le moût, et ce uniquement sous forme dissoute, ce qui évite les pertes d'éthanol par évaporation.

L'utilisation du circuit 5 extérieur au fermenteur apporte des avantages de commodité, par exemple pour l'entretien, mais suppose une fluidité suffisante du moût. Dans certains cas (par exemple fermentation des vins rouges), il sera préférable de disposer les fibres de perméation directement dans le fermenteur 1, ou de faire appel à un dispositif de filtration à l'entrée du circuit 5.

Des essais comparatifs ont été effectués dans le cas d'un vin blanc, d'une part avec une installation suivant l'invention, d'autre part avec un fermenteur témoin oxygéné par la technique classique de remontage du moût. Ces essais ont montré une meilleure tolérance à l'éthanol des levures dans le fermenteur oxygéné conformément à l'invention, et une consommation spécifique de glucose nettement accrue, au moins pour les concentrations en éthanol de 10% Vol et 11% Vol :

EP 0 334 728 B1

| Concentration en éthanol | Consommation spécifique de glucose grammes glucose/gramme levure.heure | |
|---|---|---|
| | Fermenteur témoin | Fermenteur oxygénation contrôlée |
| 9% Vol | 0,34 | 0,35 |
| 10% Vol | 0,22 | 0,26 |
| 11% Vol | 0,09 | 0,13 |

Il résulte de ces essais que l'invention permet d'augmenter l'activité fermentaire, c'est-à-dire les vitesses de fermentation. D'autre part, les analyses chromatographiques effectuées ont permis de constater l'absence de composés oxydés (acétaldéhyde, acide acétique,...) qui auraient pu apparaître en cas de suroxygénation du moût.

**Revendications**

1. Procédé d'oxygénation contrôlée d'un moût de fermentation alcoolique, caractérisé en ce qu'on met le moût (2) en contact avec une face d'une membrane perméable à l'oxygène; on met l'autre face de cette membrane en contact avec un gaz contenant de l'oxygène sous une pression partielle supérieure à la pression partielle en oxygène du liquide; on maintient constant le rapport de la pression du gaz à la pression du moût; et l'on régule l'oxygénation du moût en faisant varier la teneur en oxygène du gaz.

2. Procédé suivant la revendication 1, caractérisé en ce qu'on maintient la pression du gaz au-dessous de la valeur qui provoque l'apparition de bulles au sein du moût.

3. Procédé suivant l'une des revendications 1 et 2, caractérisé en ce qu'on mesure la concentration en oxygène dissous dans le moût, et on fait varier la teneur en oxygène du gaz de façon à maintenir cette concentration au voisinage d'une valeur de consigne.

4. Procédé suivant l'une des revendications 1 à 3, caractérisé en ce qu'on utilise comme gaz un mélange d'oxygène et d'un gaz neutre, notamment un mélange oxygène/azote.

5. Installation de fermentation alcoolique, du type comprenant un fermenteur (1) contenant un moût de fermentation (2), et des moyens d'oxygénation de ce moût, caractérisée en ce que les moyens d'oxygénation comprennent un perméateur (4) comportant une membrane perméable à l'oxygène; des moyens (5) pour mettre une face de la membrane au contact du moût; et des moyens (10 à 15) pour amener un gaz contenant de l'oxygène au contact de l'autre face de cette membrane, ces moyens d'amenée de gaz (10 à 15) comprenant une source d'un gaz neutre, notamment d'azote, une source d'oxygène, des moyens (13 à 15) de dosage et de mélange du gaz neutre et de l'oxygène, et un détendeur (11) situé en aval des moyens de dosage et de mélange.

6. Installation suivant la revendication 5, caractérisée en ce qu'elle comprend des moyens (18) de mesure de la concentration en oxygène dissous du moût, commandant lesdits moyens de dosage et de mélange (13 à 15).

7. Installation suivant l'une des revendications 5 et 6, caractérisée en ce que le perméateur (2) est du type à fibres creuses, les moyens d'amenée de gaz (10 à 15) communicant avec l'intérieur des fibres.

8. Installation suivant l'une quelconque des revendications 5 à 7, caractérisée en ce qu'elle comprend un circuit (5) de circulation du moût (2) entre le fermenteur (1) et le perméateur (4), ce circuit comportant une pompe (17).

4

**Patentansprüche**

1. Verfahren zur gesteuerten Oxygenierung einer alkoholischen Gärmaische, dadurch gekennzeichnet, daß die Maische (2) mit einer Seite einer sauerstoffdurchlässigen Membran in Kontakt gebracht wird, daß die andere Seite dieser Membran mit einem Gas in Kontakt gebracht wird, das Sauerstoff mit einem Partialdruck enthält, der höher als der Sauerstoffpartialdruck der Flüssigkeit ist, daß das Verhältnis des Gasdrucks zum Druck der Maische konstant gehalten wird, und daß die Oxygenierung der Maische dadurch reguliert wird, daß der Sauerstoffgehalt des Gases verändert wird.

2. Verfahren nach Anspruch 1, dadurch gekennzeichnet, daß der Gasdruck unterhalb eines Wertes gehalten wird, bei dem Blasen in der Maische auftreten.

3. Verfahren nach Anspruch 1 oder 2, dadurch gekennzeichnet, daß die Konzentration an in der Maische gelöstem Sauerstoff gemessen wird und der Sauerstoffgehalt des Gases derart variiert wird, daß diese Konzentration in der Nähe eines Sollwertes bleibt.

4. Verfahren nach einem der Ansprüche 1 bis 3, dadurch gekennzeichnet, daß als Gas eine Mischung aus Sauerstoff und einem neutralen Gas verwendet wird, insbesondere eine Mischung Sauerstoff/Stickstoff.

5. Anlage für die Alkoholgärung mit einem eine Gärmaische (2) enthaltenden Fermenter (1) und Mitteln zur Oxygenierung dieser Maische, dadurch gekennzeichnet, daß die Mittel zur Oxygenierung einen Permeator (4) aufweisen, der eine sauerstoffdurchlässige Membran, Mittel (5) zum Inkontaktbringen der Maische mit einer Seite der Membran und Mittel (10 bis 15) zum Zuführen eines Sauerstoff enthaltenden Gases zur anderen Seite dieser Membran enthält, wobei diese Mittel zum Zuführen von Gas (10 bis 15) eine Quelle eines neutralen Gases, insbesondere Stickstoff, eine Sauerstoffquelle, Mittel (13 bis 15) zum Dosieren und zum Mischen des neutralen Gases mit dem Sauerstoff sowie ein Druckminderventil (11) aufweisen, das den Mitteln zum Dosieren und zum Mischen nachgeschaltet ist.

6. Anlage nach Anspruch 5, dadurch gekennzeichnet, daß sie Mittel (18) zum Messen der Konzentration an gelöstem Sauerstoff der Maische aufweist, die die Mittel (13 bis 15) zum Dosieren und zum Mischen steuern.

7. Anlage nach Anspruch 5 oder 6, dadurch gekennzeichnet, daß der Permeator (4) Hohlfasern enthält und die Mittel zum Zuführen von Gas (10 bis 15) mit dem Inneren der Fasern in Verbindung stehen.

8. Anlage nach einem der Ansprüche 5 bis 7, dadurch gekennzeichnet, daß sie einen Kreis (5) für die Zirkulation der Maische (2) zwischen dem Fermenter (1) und dem Permeator (4) enthält, wobei dieser Kreis eine Pumpe (17) aufweist.

**Claims**

1. Process for the controlled oxygenation of an alcoholic fermentation must, characterised in that the must (2) is placed in contact with a surface of a membrane permeable to oxygen; the other surface of this membrane is placed in contact with a gas containing oxygen under a partial pressure greater than the partial oxygen pressure of the liquid; the ratio of the pressure of the gas to the pressure of the must is kept constant; and the oxygenation of the must is regulated by varying the oxygen content of the gas.

2. Process according to claim 1, characterised in that the gas pressure is maintained below the value which causes bubbles to appear within the must.

3. Process according to one of claims 1 and 2, characterised in that the concentration of oxygen dissolved in the must is measured and the oxygen content of the gas is varied so as to maintain this concentration in the region of a reference value.

4. Process according to one of claims 1 to 3, characterised in that, as the gas, a mixture of oxygen and a neutral gas is used, in particular a mixture of oxygen and nitrogen.

5. Installation for alcoholic fermentation, of the type comprising a fermenter (1) containing a fermentation must (2) and means for the oxygenation of this must, characterised in that the oxygenation means comprise a permeator (4) comprising a membrane permeable to oxygen; means (5) for placing a surface of the membrane in contact with the must; and means (10 to 15) for bringing a gas containing oxygen into contact with the other surface of this membrane, these gas supply means (10 to 15) comprising a source of neutral gas, in particular nitrogen, a source of oxygen, means (13 to 15) for metering and mixing neutral gas and oxygen, and a pressure reducing valve (11) situated downstream from the means for metering and mixing.

6. Installation according to claim 5, characterised in that it comprises means (18) for measuring the dissolved oxygen concentration of the must, controlling the said metering and mixing means (13 to 15).

7. Installation according to one of claims 5 and 6, characterised in that the permeator (2) is of the type with hollow fibres, the gas supply means (10 to 15) being in communication with the inside of the fibres.

8. Installation according to any one of claims 5 to 7, characterised in that it comprises a circuit (5) for the circulation of the must (2) between the fermenter (1) and the permeator (4), this circuit comprising a pump (17).